# EUROPEAN PATENT SPECIFICATION

(11) **EP 4 235 154 B1**
(45) Date of publication and mention of the grant of the patent: **23.09.2026**
(21) Application number: 22163923.0
(22) Date of filing: 23.03.2022
(51) Int. Cl.: G01N 21/64, G02B 21/16, G02B 21/36

(54) **BIOMARKER IDENTIFICATION METHOD**
VERFAHREN ZUR IDENTIFIZIERUNG VON BIOMARKERN
PROCÉDÉ D'IDENTIFICATION DE BIOMARQUEUR

(30) Priority: 25.02.2022 KR 20220025049
(43) Date of publication of application: 30.08.2023
(73) Proprietor: Stanos Inc., Seoul 08511 (KR)
(72) Inventor: LEE, Jong Jin, 28124 Cheongju-si, Chungcheongbuk-do (KR)
(74) Representative: Prüfer & Partner mbB Patentanwälte · Rechtsanwälte

(56) References cited:
- WO-A1-2007/011549
- WO-A2-2018/085531
- US-A1- 2017 176 338
- BOGDANOV V ET AL: "PARALLEL, CONFOCAL, AND COMPLETE SPECTRUM IMAGER FOR FLUORESCENT DETECTION OF HIGH-DENSITY MICROARRAY", PROCEEDINGS OF SPIE, IEEE, US, vol. 3605, 24 January 1999 (1999-01-24), pages 298 - 307, XP000917120, ISBN: 978-1-62841-730-2, DOI: 10.1117/12.347576
- HEIDER EMILY C. ET AL: "Identification of Single Fluorescent Labels Using Spectroscopic Microscopy", APPLIED SPECTROSCOPY., vol. 64, no. 1, 1 January 2010 (2010-01-01), US, pages 37 - 45, XP055958133, ISSN: 0003-7028, DOI: 10.1366/000370210790572034

## Description

### BACKGROUND

### Technical Field

The present disclosure relates to a fluorescence microscope for identifying a biomarker and a biomarker identification method using the same, and more particularly, to a fluorescence microscope for identifying a biomarker capable of easily identifying a type of biomarker through fluorescence analysis and a biomarker identification method using the same.

### Background Art

A fluorescence microscope is an optical microscope for imaging using fluorescence, and may detect and image long-wavelength light in which light absorbed by a fluorescent material is emitted in the form of fluorescence when a specific wavelength absorbed by the fluorescent material present in a sample is illuminated.

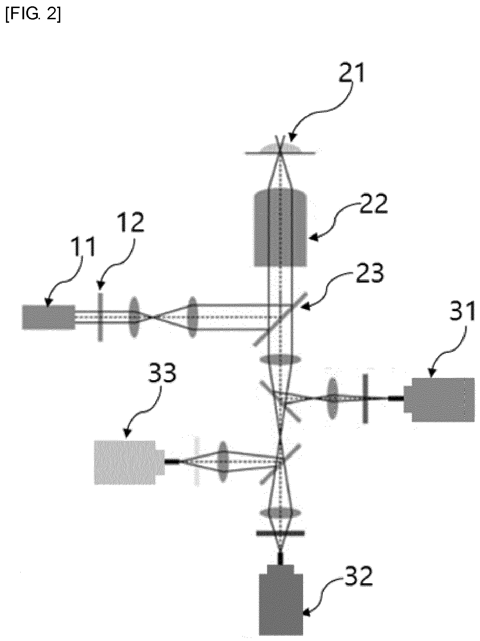

The fluorescence microscope detects emitted light that is much weaker than light illuminated using a wavelength-specific filter. The fluorescence microscope consists of a light source using xenon, mercury, an LED and a laser, an excitation filter, a dichroic mirror, and an emission filter. The light emitted from the light source passes through only a wavelength that can be absorbed by the fluorescent material through the excitation filter, and illuminates the sample through the dichroic mirror. The fluorescent material in the sample absorbs the specific-wavelength light and emits long-wavelength light in the form of fluorescence, and the emitted light is not reflected to the dichroic mirror, but is detected by a detector. In the case of a multicolor fluorescence microscope, it is possible to image various colors of fluorescent materials present in the sample by using the excitation filter and the dichroic mirror corresponding to each color (see FIG. 2).

Such a fluorescence microscope is used to image intracellular organelles, proteins, and the like, and includes a confocal microscope, a total internal reflection fluorescence microscope, and the like.

In a conventional fluorescence microscope, monochromatic light matching an absorption wavelength of a fluorophore attached to a sample placed on a substrate is selected from white light through a first optical filter 12, a path of the selected monochromatic light having the absorption wavelength is adjusted through a dichroic mirror 23 and irradiated to the sample through an objective lens 22, and light matching a emission wavelength of the fluorophore of the sample is selected from the light generated by the fluorophore of the sample that has passed through the objective lens and the dichroic mirror through a second optical filter to be provided to the detection unit. On the other hand, the detection unit is implemented with a tube lens, cameras 31, 32, and 33 such as a CCD or a CMOS, and detects and displays the emission wavelength of the fluorophore attached to the sample to observe the shape of the sample or the fluorescence emitted by the sample (see FIG. 2).

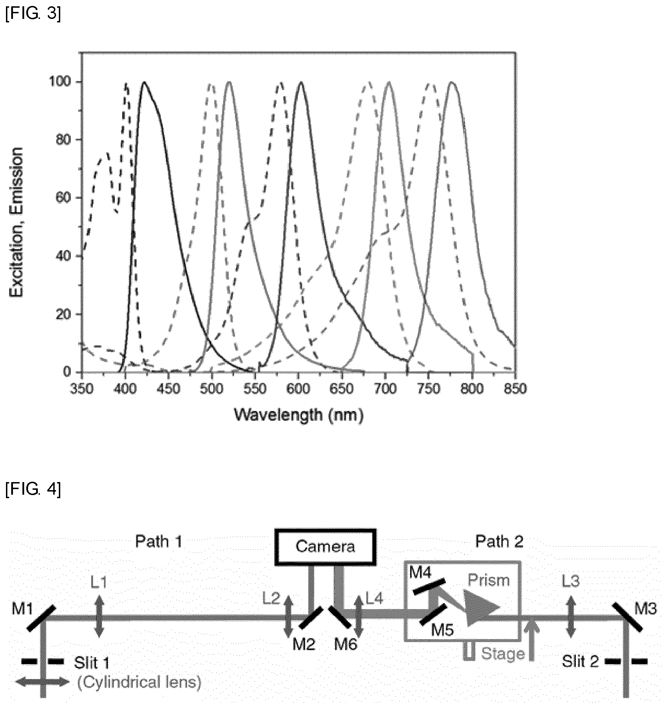

However, since the conventional fluorescence microscope has a structure in which a single fluorescence image is obtained according to light irradiated to the sample, there is a disadvantage that it is necessary to use various types of fluorescent dyes to identify various types of samples, and there is a need for various wavelengths of light sources and cameras required for excitation and detection of each fluorescent dye (see FIG. 3).

In particular, in order to clearly identify between these samples, since it is preferred that fluorescence spectra emitted by each fluorophore do not overlap with each other, there are problems that only 2 to 5 types of fluorophores may be used simultaneously so that the types of samples to be identified are limited, and as the same quantity of light sources as the fluorophores is required, the size of the device increases, it becomes expensive, and the detection time increases (see FIG. 3).

In order to solve this problem, a technique has been developed to utilize the fluorescence spectrum emitted from the sample using a prism. It identifies each fluorophore by comparing a position of an original fluorescence with relative positions of the fluorescence dispersed by the prism (see FIG. 4). In the case of this technology, since the type of fluorescence is identified by analyzing a relative position, various types of fluorophores with overlapped absorption spectrum and emission spectrum may be used to enable analysis of various types of samples and greatly reduce the number of light source units. However, in this technology, since it is required to compare the positions, it is necessary to install a detection unit for original fluorescence and an additional detection unit for fluorescence dispersed by the prism, respectively, and the emitted fluorescence needs to be divided along two paths, and thus, there is a disadvantage that the detection area is reduced and sensitivity is lowered (see FIG. 4).

Therefore, there is a need to manufacture a fluorescence microscope capable of simultaneously analyzing various types of samples while minimizing changes in the detection area and sensitivity during sample analysis using fluorescence.

### [Prior Art Documents]

### [Patent Documents]

Korean Patent No. 10-0942195
Korean Patent No. 10-2290325

The further patent document WO 2007/011549 A1 discloses fluorescence spectral imaging for identifying biomolecules, wherein fluorescence spectra from respective examination sites of a microplate are acquired. An emission filter is used for truncating each emission spectrum at both ends to prevent crosstalk.

The above information disclosed in this Background section is only for enhancement of understanding of the background of the invention and therefore it may contain information that does not form the prior art that is already known in this country to a person of ordinary skill in the art.

### SUMMARY OF THE DISCLOSURE

In order to solve the problems described above, the present invention provides biomarker identification method as defined in claim 1.

A non-claimed aspect of the present disclosure provides a fluorescence microscope capable of identifying a biomarker in a fluorescence microscope for analyzing fluorescence emitted from a sample, wherein a detection unit of the fluorescence microscope is provided with an identification filter that blocks some of fluorescence spectra incident to the detection unit.

In an embodiment, the identification filter may block at least one of some of fluorescence spectra having short-wavelength emission peaks in the fluorescence emitted from the sample; and some of fluorescence spectra having long-wavelength emission peaks in the fluorescence emitted from the sample.

In an embodiment, the identification filter may block wavelengths of a peak wavelength or shorter of the fluorescence spectra having the short-wavelength emission peaks in the fluorescence emitted from the sample; and wavelengths of a peak wavelength or longer of the fluorescence spectra having the long-wavelength emission peaks in the fluorescence emitted from the sample.

In an embodiment, the fluorescence emitted from the sample may have 2 to 100 emission peaks, and some or all of the emission spectra having each peak overlap with each other.

In an embodiment, fluorophores included in the sample may be simultaneously emitted with one or more excitation light.

In an embodiment, the sample may include 2 to 100 types of fluorophores.

In an embodiment, the sample may include a capture probe attached to a substrate; a biomarker bound to the capture probe; and a detection probe bound to the biomarker is labeled with a fluorophore, wherein 2 to 100 types of detection probes may be present on the surface of the substrate.

In an embodiment, the detection probe may be attached with at least one type of fluorophores, therefore the biomarker bound to the detection probe may have one or more emission peaks.

In an embodiment, the detection unit may include a spectroscopic means for spectralizing the fluorescence emitted from the sample.

In an embodiment, the identification filter may be provided in front of the spectroscopic means.

In an embodiment, the fluorescence dispersed by the spectroscopic means may be observed in an elliptical shape with one or both cut sides.

The invention provides a biomarker identification method including steps of preparing a sample labeled with a fluorophore; positioning the sample on an objective unit of a fluorescence microscope; illuminating the sample using a light source; blocking some of fluorescence spectra emitted from the sample using an identification filter; dispersing the fluorescence with the spectroscopic means; and identifying a type of sample using the observed shape of the spectrum.

In the invention, the identifying of the type of sample includes recognizing a blocked point of the spectrum of the fluorescent light; analyzing a relative position and length of the fluorescence spectrum from the blocked point; and identifying a type of sample using the relative position and length of the fluorescence spectrum.

In an embodiment, the sample labeled with the fluorophore may be 2 to 100 types of biomarkers labeled with different fluorophores.

According to the present disclosure, since the fluorescence microscope for identifying the biomarker may identify a type of biomarker using a difference in emission spectrum, the fluorescence microscope may be widely used for rapid and accurate identification of a plurality of biomarkers.

Further, according to the present disclosure, since the type of fluorescence may be identified by blocking some of the spectrum of the fluorescence, it is possible to provide a fluorescence microscope with improved detection area and sensitivity compared to an existing analysis method using a difference in emission wavelength.

In addition, according to the present disclosure, since a shape thereof is identified by blocking some of fluorescence spectrum wavelengths and then dispersing the fluorescence spectrum, it is possible to identify the shape even when fluorescence having a similar peak emission wavelength is used. Accordingly, the number of excitation light may be reduced, and various types of fluorophores may be used at the same time.

### BRIEF DESCRIPTION OF THE DRAWINGS

FIG. 1 is a diagram illustrating a structure of a fluorescence microscope capable of identifying a biomarker according to an embodiment of the present disclosure.
FIG. 2 is a diagram illustrating a structure of a conventional fluorescence microscope.
FIG. 3 is a diagram illustrating an absorption spectrum(dashed line) and an emission spectrum(solid line) of a fluorophore used in a conventional fluorescence microscope, respectively.
FIG. 4 is a diagram illustrating a structure of a conventional fluorescence microscope using a spectroscopic means.
FIG. 5 is a photograph showing a sample observed by the conventional fluorescence microscope using the spectroscopic means.
FIG. 6 is a diagram illustrating changes in an emission spectrum of a fluorophore and an emission spectrum passing through an identification filter according to an embodiment of the present disclosure.
FIG. 7 is a diagram illustrating a state in which an emission spectrum by an identification filter is observed in the case of using a plurality of fluorophores according to an embodiment of the present disclosure.
FIG. 8 is a diagram illustrating a state in which an emission spectrum is observed in the case of using different types of identification filters according to an embodiment of the present disclosure.
FIG. 9 is a diagram illustrating an absorption spectrum and an emission spectrum of a fluorophore according to an embodiment of the present disclosure.
FIG. 10 is a diagram illustrating a step of preparing a sample according to an embodiment of the present disclosure.
FIG. 11 is a diagram illustrating observing the fluorescence of the sample according to an embodiment of the present disclosure, wherein FIG. 11A illustrates before spectroscopy and FIG. 11B illustrates after spectroscopy after passing through identification filters.
FIG. 12 is a diagram illustrating an emission spectrum of a fluorophore according to an embodiment of the present disclosure.
FIG. 13 is a diagram illustrating an absorption spectrum of a fluorophore according to an embodiment of the present disclosure.
FIG. 14 is a diagram illustrating fluorescence without using an identification filter and a spectroscopic according to an embodiment of the present disclosure.
FIG. 15 is a diagram illustrating observing fluorescence after using only a spectroscopic according to an embodiment of the present disclosure.
FIG. 16 is a diagram illustrating observing fluorescence after using an identification filter and a spectroscopic means according to an embodiment of the present disclosure.
FIG. 17 is a diagram illustrating a spectral distribution of each fluorescence after using an identification filter and a spectroscopic means according to an embodiment of the present disclosure.

### DETAILED DESCRIPTION

Hereinafter, preferred embodiments of the present disclosure will be described in detail. In describing the present disclosure, a detailed description of related known technologies will be omitted if it is determined that the detailed description makes the gist of the present disclosure unclear. Throughout the specification, singular expressions should be understood to include plural expressions unless otherwise clearly indicated in contexts. In the present specification, it should be understood that term "including" or "having" indicates that a feature, a number, a step, an operation, a component, a part or a combination thereof to be described is present, but does not exclude a possibility of presence or addition of one or more other features, numbers, steps, operations, components, parts or combinations thereof, in advance. Further, in performing methods or manufacturing methods, respective processes of configuring the method may be performed differently from a specified order unless otherwise describing a specific order in the context. That is, the respective processes may be performed similarly to the specified order, performed substantially simultaneously, and performed in an opposite order.

The present disclosure may use various modifications and have various embodiments, so specific embodiments will be illustrated and described in detail in the detailed description.

The techniques disclosed herein are not limited to the embodiments described herein and may also be embodied in other forms. Only the embodiments introduced herein are provided to allow the disclosed content to be thorough and complete, and to allow the technical ideas of the technology to be sufficiently delivered to those skilled in the art. In order to clearly express the components of each device in the drawings, the sizes such as widths and thicknesses of the components are slightly enlarged. In the description of the drawings as a whole, it has been described from an observer's aspect, and when it is mentioned that an element is positioned on the other element, the element may be positioned directly on the other element or an additional element may be interposed between the elements. In addition, on a plurality of drawings, the same reference numerals refer to substantially the same elements as each other.

In the present specification, the term 'and/or' includes a combination of a plurality of disclosed items or any item of the plurality of disclosed items. In the present specification, 'A or B' may include 'A', 'B', or 'both A and B'.

The present disclosure relates to a (non-claimed) fluorescence microscope capable of identifying a biomarker in a fluorescence microscope for analyzing fluorescence emitted from a sample, in which a detection unit of the fluorescence microscope is provided with an identification filter that blocks some of fluorescence spectra incident to the detection unit.

The fluorescence microscope used in the present disclosure is a microscope capable of observing fluorescence generated from the sample, and may be largely constituted by an objective unit 100, a light source unit 200, and a detection unit 300.

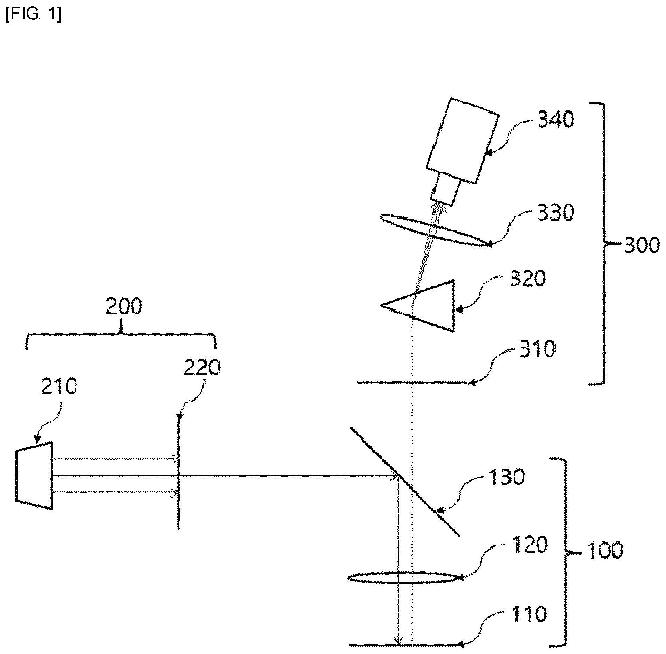

The objective unit 100 is a part on which a sample 110 is placed and fixed for easy observation, includes a fixing means for fixing the sample 110, and may be installed with an objective lens 120 for collecting/enlarging the fluorescence emitted from the sample 110 while illuminating the sample 110. In addition, a dichroic mirror 130 is installed to reflect the excitation light supplied from the light source unit 200 and supply the reflected excitation light in the direction of the sample, and transmit the fluorescence generated from the sample 110 to the detection unit (see FIG. 1).

The dichroic mirror 130 is a kind of mirror that reflects light of a specific range of wavelength and transmits light of the other range of wavelength, and in the case of the present disclosure, may simultaneously perform both the reflection of the excitation light and the transmission of the fluorescence.

The light source unit 200 is a part that generates excitation light to excite the fluorophore of the sample, and may be provided with an excitation light filter 220 to supply only the excitation light required for the sample. The excitation filter 220 may be a filter that transmits only light of a wavelength capable of exciting the fluorophore labeled on the sample in the light supplied from the light source 210 of the light source unit. In addition, the excitation filter 220 is preferably attached in a replaceable manner to form excitation light of various wavelengths.

The light source 210 may be used without limitation as long as it is light capable of including the wavelength of the excitation light, but preferably, a white light emitting device having high color rendering may be used. The white light emitting device having the high color rendering has a smooth emission spectrum distributed over the entire visible light region, and emits light so that this spectrum extends to a ultraviolet region, which is often used as excitation light to have an emission pattern similar to sunlight. In the case of the high color rendering light emitting device, since light is emitted by mixing various luminants and fluorophores, excitation light of a desired spectrum may be formed using the excitation filter 220. In particular, in the case of light sources used in the related art, only light with a narrow spectrum is supplied, and when excitation light other than the spectrum supplied from the light source is required, the light source itself needs to be exchanged. In the case of using the white light emitting device having the high color rendering as described above, desired excitation light may be formed only by exchanging the excitation filter 220 without replacing the light source.

In addition to the white light emitting device, a laser emitting monochromatic light may be used as a light source, and in this case, it is preferred that the monochromatic light emitted by the laser may simultaneously excite the fluorophore. The laser may have a higher luminous intensity than a light source using the light emitting device, and may emit monochromatic light having a narrow spectrum band. Accordingly, since the fluorophore may be excited more brightly, such a laser light source may be used if the fluorophores may use the same excitation light. However, when a wide spectrum is required to excite the fluorophores, it is preferred to use the white light emitting device having the high color rendering, so that it is preferred to select and use an appropriate light source according to conditions of each experiment.

The detection unit 300 is a part installed to observe the fluorescence generated from the sample and includes a tube lens 330 to facilitate the observation of the fluorescence. However, if finite conjugated objective lens is used, the tube lens can be omitted.

In the case of a fluorescence microscope used in the related art (see FIG. 2), since the type of fluorescence is determined by simply measuring a peak wavelength of fluorescence, in the case of using a fluorescence having the similar peak wavelength, there is a disadvantage that a type of fluorescence, and a type of sample attached with the fluorophore cannot be identified. In addition, since a plurality of cameras 31, 32, and 33 (FIG. 2) according to each color need to be used to detect the fluorescence generated by the fluorophore, there is a problem that the size and cost of the device increase. In addition, in the case of fluorophores having different peak wavelengths as described above, absorption spectra are also different (see FIG. 3), so that there is a problem that various types of excitation light need to be used.

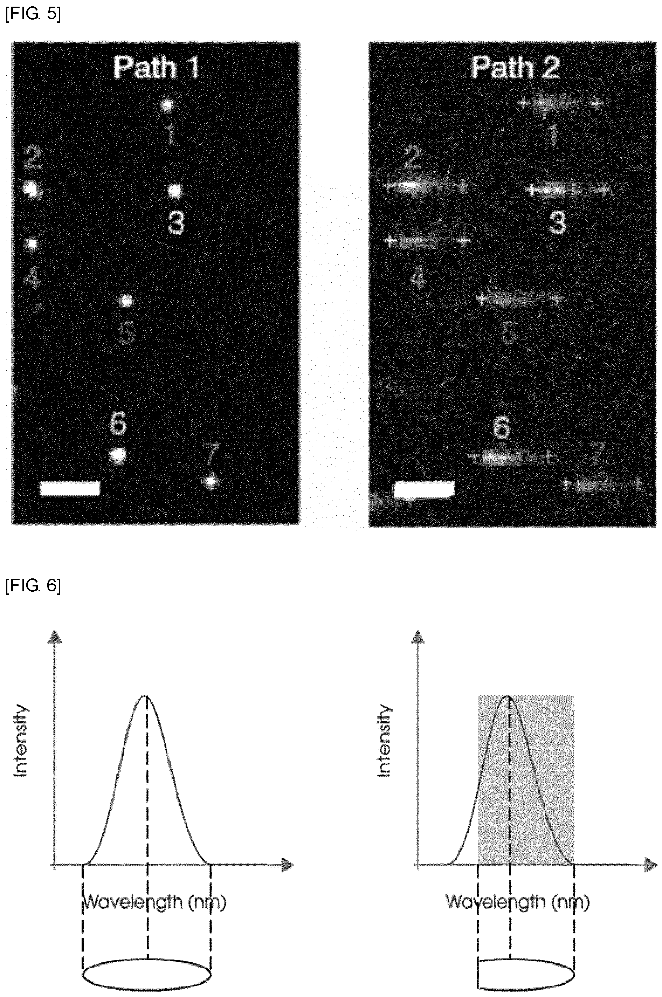

In order to solve this problem, a technique of dispersing the fluorescence generated from the sample and then identifying the fluorescence using a difference between an original position and a position of the dispersed light has been used (see FIG. 4). In this technique, since the fluorescence is detected using a position difference rather than a color of fluorescence, one detector may be used, and may be used even when the overlapping between fluorescence spectra is severe. As illustrated in FIG. 5, in this technology, two phases are combined to confirm a light emitting point (path 1) and identify the spectrum (path 2), and the type of sample is identified using a relative position difference between the light emitting point and the spectrum. However, there is a problem in that the detection area is reduced and the sensitivity is lowered at the same time because the fluorescence signal needs to be divided into two paths for determining the light emitting point of fluorescence (path 1) and a path for spectrum (path 2).

Accordingly, in the present disclosure, in order to determine the type of fluorescence without dividing the light path, an identification filter that blocks some of fluorescence spectra is installed in the detection unit to identify the type of fluorescence.

The fluorophore labeled on the sample 110 emits fluorescence due to the excitation light supplied from the light source unit 200, and the fluorescence passes through the dichroic mirror 130 to be supplied to the detection unit 300. In addition, a spectroscopic means 320 is installed inside the detection unit 300, and the fluorescence that has passed through the spectroscopic means 320 may be dispersed by a difference in wavelength to be observed in an elliptical shape (see the left figure of FIG. 6).

In the present disclosure, since some wavelengths of the fluorescence are blocked using the identification filter 310, one or both sides of the elliptical shape may be observed in a cut form (see the right figure of FIG. 6). That is, the type of fluorophore may be identified by determining the relative position and length of the elliptically dispersed fluorescence from the blocked point.

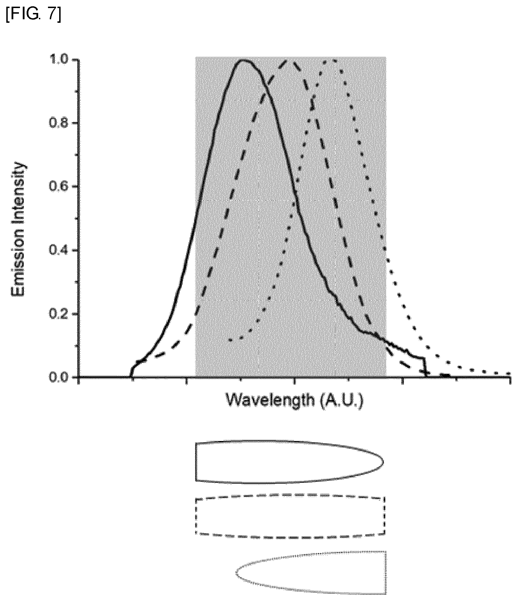

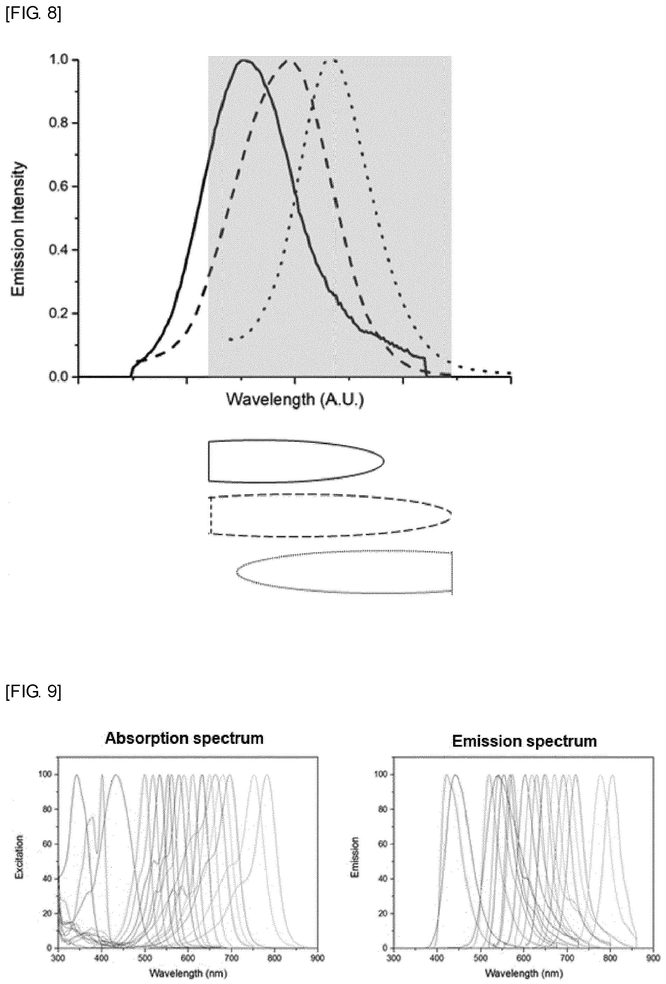

The fluorescence emitted from the sample 110 may have a different spectrum depending on a type thereof. Accordingly, in the case of passing through the identification filter that blocks predetermined wavelengths, when the fluorescence is dispersed, the fluorescence may be observed in a part of the elliptical shape that is in a cut form (see right figure of FIG. 6), and the cut position may be determined by a relative combination of the spectrum distribution of the fluorescence and the blocking wavelength of the identification filter (see FIGS. 7 and 8).

For example, in the case of red fluorescence having a spectral distribution of 470 nm to 670 nm and an emission peak of 530 nm, when the red fluorescence passes through an identification filter that blocks wavelengths of 500 nm and shorter, elliptical fluorescence with a cut part of the left side may be observed (short wavelengths of light are dispersed to the left). In addition, in the case of using an identification filter that blocks wavelengths of 600 nm and longer, the red fluorescence may be observed as a shape in which the right side of the ellipse is cut. That is, when an appropriate identification filter is used, an ellipse having a different shape and size may be observed depending on a type of fluorescence, which means that the fluorescence may be identified only by appropriately selecting and using the identification filter.

In addition, when the spectral peaks of the fluorescence are close to each other, two types of fluorescence may be identified by a relative shape difference of the elliptical fluorescence remaining from the cut portion, which means that even when a plurality of fluorophores having adjacent emission peaks are used simultaneously, the two types of fluorescence may be identified (see FIG. 7).

In addition, to facilitate the identification as described above, the identification filter 310 may block at least one of portions of a fluorescence spectrum having an emission peak of a shorter wavelength in the fluorescence emitted from the sample; and a portion of a fluorescence spectrum having an emission peak of a long wavelength in the fluorescence emitted from the sample.

In general, in the case of the emission spectrum, since the brightest fluorescence is observed in the emission peak band, it is preferred to block a part of the emission spectrum except for the peak band. However, since the fluorescence may be observed due to the remaining portion even when the portion including the emission peak band is blocked, it is preferred to use an identification filter capable of blocking a part of the fluorescence spectrum as described above.

In addition, to facilitate the identification as described above, the identification filter 310 may block wavelengths of a peak wavelength or shorter of a fluorescence spectrum having an emission peak of a short wavelength in the fluorescence emitted from the sample; and wavelengths of a peak wavelength or longer of a fluorescence spectrum having an emission peak of a long wavelength in the fluorescence emitted from the sample. At this time, the fluorescence spectrum having the emission peak of the short wavelength and the fluorescence spectrum having the emission peak of the long wavelength are observed by removing the wavelengths shorter or longer than the peak wavelength, and in the case of the remaining fluorescence, the peak wavelength is located between the two types of fluorescence (see FIG. 7). Accordingly, the fluorescence spectrum having the emission peak of the short wavelength and the fluorescence spectrum having the emission peak of the long wavelength may act as a kind of reference point, and based on this, the fluorescence may be identified by measuring a difference between the shape of the ellipse and the relative position with the reference points.

In addition, in this case, the fluorescence having the emission peak of the short wavelength may preferably be fluorescence having an emission peak of the shortest wavelength, and the fluorescence having the emission peak of the long wavelength is preferably fluorescence having the emission peak of the longest wavelength. By blocking the wavelength shorter or longer than the peak wavelength of fluorescence having the shortest emission peak or the longest emission peak, it is possible to maximize the effect of the identification filter as described above.

The fluorescence emitted from the sample may have 2 to 100 emission peaks, and some or all of the emission spectra having each peak may overlap with each other to be emitted. As described above, in the case of the present disclosure, a plurality of fluorescence may be identified, and it is preferable that the fluorescence is observed in an elliptical shape with one or both cut sides by the identification filter. Therefore, it is preferable that some or all of the emission spectra of the fluorescence overlap with each other (see FIG. 7), and in this case, a part of the fluorescence is blocked by the identification filter to be identified. In addition, when the emission spectra overlap with each other as described above, absorption spectra generally also overlap (see FIG. 9), so that all fluorophores may be excited even when one excitation light is used in the light source unit. Through this, in the case of the present disclosure, the fluorophores of the entire sample may be excited by using not only a single light source, but also excitation light having one wavelength, and may be identified using the identification filter, thereby greatly improving convenience as compared with an existing fluorescence analysis method.

In addition, when more fluorescence is simultaneously identified, a plurality of excitation light may be used because all of the fluorophores cannot be excited with one excitation light. As described above, it is most preferred to excite all of the fluorophores with one excitation light, but when a plurality of detections are required, the number of fluorophores is inevitably increased, and in this case, all of the fluorophores may not be excited with one excitation light. Therefore, in this case, it is preferred to use a plurality of excitation light. In addition, since peaks of the absorption wavelength are different from each other according to each fluorescence, even when all fluorescence may be excited using one excitation light, a plurality of excitation light may be simultaneously or sequentially supplied to obtain an optimal image. In this case, one or more additional dichroic filters are required to block the transmission of excitation light to the detection unit. These filters may block some of the fluorescence spectra and the resulting blocked points can be used as additional reference points to identify fluorophores.

In addition, as described above, according to the present disclosure, even in the case of using fluorophores having similar emission peaks, as the fluorophores may be identified to identify fluorophores having 2 to 100 emission peaks at the same time. Therefore, in the case of the present disclosure, various types of samples may be identified at the same time.

The sample may include: a capture probe attached to a substrate; a biomarker bound to the capture probe; and a detection probe bound to the biomarker is labeled with a fluorophore, wherein 2 to 100 types of detection probes may be present on the surface of the substrate.

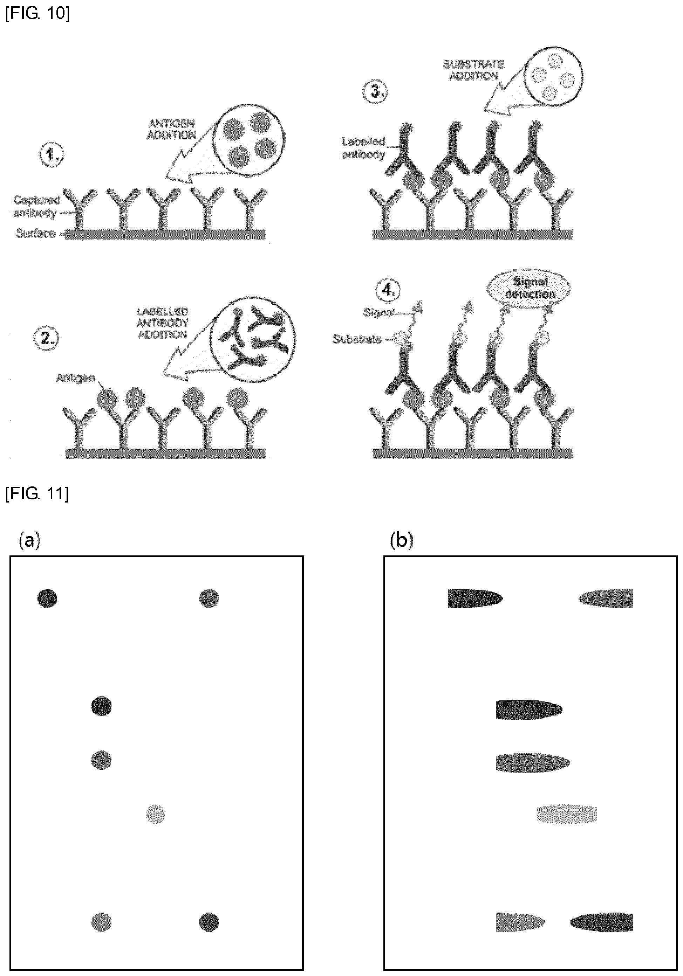

In the case of the sample, a sample used for general fluorescence analysis may be used, and in particular, may be used for an immunological analysis method of a biomarker used in the related art (see FIG. 10).

In detail, different capture probes are attached to the substrate, and the capture probes may capture a specific biomarker. In this case, when a body fluid containing the biomarker is supplied onto the substrate, the biomarker may be bound to the capture probe attached to the surface of the substrate.

In addition, the detection probe may be labeled with one or more fluorophores, therefore, the biomarker bound to the detection probe may have one or more emission peaks.

After the attachment of the biomarker is completed as described above, a detection probe capable of specifically binding to each biomarker may be supplied to be attached to the biomarker. In this case, the detection probe may be labeled with one fluorophore to emit fluorescence having one emission peak, or may be labeled with two or more fluorophores to emit fluorescence having two or more emission peaks. In addition, since the different type of detection probe includes the same or different fluorophore as or from the other detection probes, a plurality of detection probes may be simultaneously identified or each detection probe may be independently identified.

That is, the different fluorophore or a combination of two or more fluorophores is labeled on each detection probe, and accordingly, the biomarker attached with the detection probe may emit fluorescence having a specific emission peak or two or more emission peaks.

Therefore, when the biomarker is present, a specific point on the substrate may generate fluorescence by the detection probe bound to the biomarker, and it is difficult to identify the type of fluorescence by morphologically analyzing the fluorescence in the present disclosure.

The detection unit 300 may include a spectroscopic means 320 for dispersing the fluorescence emitted from the sample. Since the fluorescence transmitted to the detection unit 300 is emitted from the fluorophore labeled on the sample 110, the fluorescence may be observed like simple circular spot. In the present disclosure, in order to analyze the fluorescence, it is preferred to disperse the fluorescence according to a spectral wavelength, so that the detection unit may be provided with the spectroscopic means 320 capable of dispersing the fluorescence.

In this case, the spectroscopic means 320 may be used without limitation as long as the spectroscopic means may disperse the fluorescence, but preferably, may use a spectrometer including a prism or a diffraction grating.

The identification filter 310 may be installed in front of the spectroscopic means 320. In the case of the fluorescence passing through the spectroscopic means 320, as described above, the fluorescence may be dispersed to have an elliptical shape, and in the present disclosure, some wavelengths of the fluorescence are blocked using the identification filter 310. The identification filter 310 may be constituted by one or more long-pass filters, short-pass filters, band-pass filters, or a combination thereof. The long-pass filter is a filter that passes through a relatively long-wavelength portion, and when the filter is used, a short-wavelength portion may be blocked. In addition, on the contrary, the short-pass filter is a filter that passes through a relatively short-wavelength portion, and when the filter is used, a long-wavelength portion may be blocked. Therefore, in the present disclosure, a combination of the long-pass filter and the short-pass filter may be used as an identification filter that passes through only a predetermined band. Separately, it is also possible to use a band-pass filter that passes through only a wavelength of a predetermined band.

The present disclosure relates to a biomarker identification method as in the invention including steps of preparing a sample labeled with a fluorophore; positioning the sample on an objective unit of a fluorescence microscope; illuminating the sample using a light source; blocking some of fluorescence spectra emitted from the sample using an identification filter; dispersing the fluorescence with the spectroscopic means; and identifying a type of sample using the observed shape of the spectrum.

The preparing of the sample labeled with the fluorophore is a step of preparing the sample by attaching a biomarker and a fluorophore to a substrate and the sample may be prepared in a similar manner to a general fluorescence analysis method. That is, in the invention, after a capture probe is attached to the substrate, the biomarker is bound to the capture probe, and a detection probe is specifically attached to the biomarker. In addition, as described above, since the detection probe is labeled with the fluorophore, it is possible to emit fluorescence having a constant spectrum by supplying the excitation light to be described below (see FIG. 10).

The probe may be used without limitation as long as the probe may be attached to the biomarker as described above, and includes an antibody, a nanobody, a variant of an antibody such as a single-chain fragment variable (scFv), an aptamer, etc.

After the sample prepared as described above is positioned in the objective unit of the fluorescence microscope, the excitation light may be supplied to the sample using the light source. The excitation light refers to light that supplies energy capable of emitting the fluorophore included in the sample, and emits a predetermined wavelength of fluorescence while some electrons of the fluorophore become in an excited state using the excitation light and electrons in the excited state return to a normal state. Accordingly, it is preferred to excite the fluorophore using appropriate excitation light according to its type. In addition, in the case of the present disclosure, as described above, the same excitation light may be used even in the case of a sample including 2 to 100 kinds of fluorophores.

As described above, when the fluorescence is emitted by the excitation light, the fluorescence may be incident to the detection unit. In this case, some of the fluorescence spectra may be blocked by the identification filter installed in the detection unit (see FIGS. 6 to 8). The fluorescence with some wavelengths blocked as described above may be dispersed through the spectroscopic means, and as described above, since the fluorescence is observed as an ellipse with one or both cut sides, it is possible to identify the type of each fluorescence and furthermore, the type of sample attached with the fluorophore.

At this time, in detail, the identifying of the type of sample includes recognizing a blocked point of the fluorescence spectrum; analyzing a relative position and length of the fluorescence spectrum from the blocked point; and identifying a type of sample using the relative position and length of the fluorescence spectrum.

The dispersed fluorescence may be observed in an elliptical shape depending on the wavelength and intensity of the fluorescence (see the left figure of FIG. 6). In detail, the fluorescence passing through the spectroscopic means may be arranged with a long length according to a wavelength because a short wavelength has a large refractive index (= refraction angle) and a long wavelength has a small refractive index (= refraction angle). In addition, a middle portion arranged as described above includes emission peaks to be observed to be brighter and thus may be observed to be relatively thicker than both ends thereof. In detail, the fluorescence passing through the spectroscopic means may be observed in an elliptical shape.

In addition, according to the present disclosure, since some of the fluorescence spectra are blocked by using the identification filter, the fluorescence may be observed in a cut form of the elliptical shape (see right figure of FIG. 6), and the cut portion, that is, a blocked point is recognized to be used as a reference point. At this time, the recognition of the blocked point may be visually identified by the user and manually designated, but since the blocked point is blocked in a straight line unlike other portions, a recognition device controlled to automatically recognize the light formed in the straight line may also be designated automatically.

After the recognition of the blocked point as described above is completed, the relative position and length of the blocked point and the dispersed fluorescence may be identified. When the identification filter blocks the short-wavelength portion of the spectrum, the fluorescence is observed in the elliptical shape with a cut left side (spectrum so that the left side has a short wavelength), and when the identification filter blocks the long-wavelength portion of the spectrum, the fluorescence may be observed in the elliptical shape with a cut right side.

Therefore, when fluorescence exists at the right of the blocked point, the emission peak of the fluorescence uses a fluorophore having a relatively short wavelength, and when fluorescence exists at the left of the blocked point, the emission peak of the fluorescence may be identified by using a fluorophore having a relatively long wavelength. In addition, when the fluorescence is observed as an elliptical shape with both cut sides, the fluorescence may be identified as using a fluorophore having an emission peak in an intermediate wavelength band (see FIG. 7).

In addition, when there are a plurality of fluorophores that form fluorescence in the same direction at the blocked point, the type of sample to which each fluorophore is labeled may be identified by measuring the total length of the fluorescence from the blocked point and the length to the thickest portion of the elliptical shape (see FIG. 8). In this case, the total length of the fluorescence represents the total wavelength band of the fluorescence spectrum that is not blocked by the identification filter, and since the thickest portion of the elliptical shape means an emission peak point generated by the fluorophore, each sample may be identified even when a blocked point is formed in the same direction by comprehensively observing the two aspects. This means that even in the case of using fluorescence having a similar wavelength as illustrated in FIG. 11, the type of fluorescence may be easily identified. As illustrated in FIG. 11A, when a fluorophore having a similar emission color is used, it may not be easy to identify the type. However, in the case of the present disclosure, as illustrated in FIG. 11B, the fluorescence is dispersed using the identification filter and the spectroscopic means, and then a part thereof is blocked to identify the type thereof.

Hereinafter, a preferred embodiment of the present disclosure will be described hereinafter with reference to the accompanying drawings so that those skilled in the art will easily implement the preferred embodiment of the present disclosure. Further, in describing the present disclosure, detailed description of associated known function or constitutions will be omitted if it is determined that they unnecessarily make the gist of the present disclosure unclear. In addition, some features presented in the drawings are enlarged, reduced, or simplified for ease of description, and the drawings and components thereof are not necessarily illustrated at appropriate ratios. However, those skilled in the art will easily understand these details.

### Examples

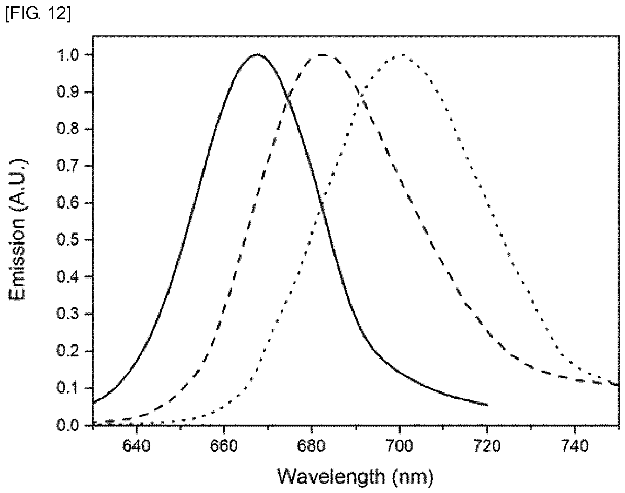

In order to verify whether three types of biomarkers CA19-9, CRP, and TTR can be identified, CF647 as a fluorophore was labeled on a detection antibody of CA19-9, CF660R as a fluorophore was labeled on a detection antibody of CRP, and CF680R as a fluorophore was labeled on a detection antibody of TTR. As illustrated in FIG. 12, each of the fluorophores represented emission spectra of a solid line (CF647), a dashed line (CF660R), and a dotted line (CF680R).

An FF01-680/42-25 filter of Semrock was used as an identification filter for blocking some of the spectra of each fluorophore. This identification filter transmitted wavelength between 657 nm and 704 nm only.

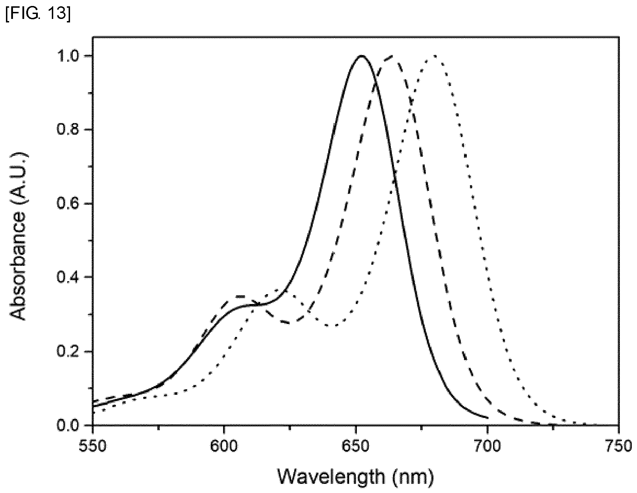

In addition, the three types of fluorophores had absorption spectra illustrated in FIG. 13. Therefore, in order to simultaneously excite the three types of fluorophores CF647, CF660R, and CF680R, a laser with a wavelength of 633 nm was used as an excitation light source.

### Experimental Example

The three types of biomarker capture antibodies of Example were attached to the surface of the substrate, the three types of biomarkers CA19-9, CRP, and TTR were injected and attached to the capture antibodies, and then the detection antibodies of the three biomarkers labeled with the fluorophores were injected to be bound to the three biomarkers.

The sample was positioned on the objective unit of the fluorescence microscope, and excitation light (laser with a wavelength of 633 nm) was supplied using a light source. The fluorescence emitted from the sample was observed.

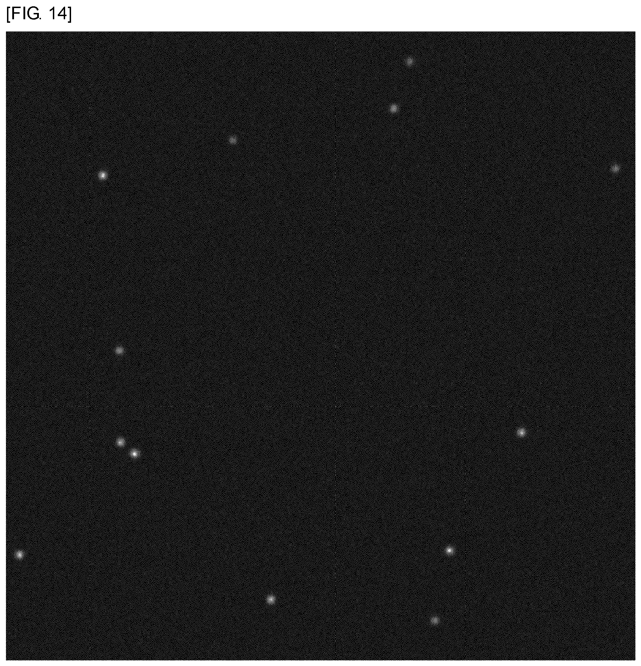

FIG. 14 is a microscopy image without using an identification filter and a spectroscopic means. It was confirmed that three types of biomarkers labeled with each fluorophores emit fluorescence, but the three types of biomarkers could not be identified.

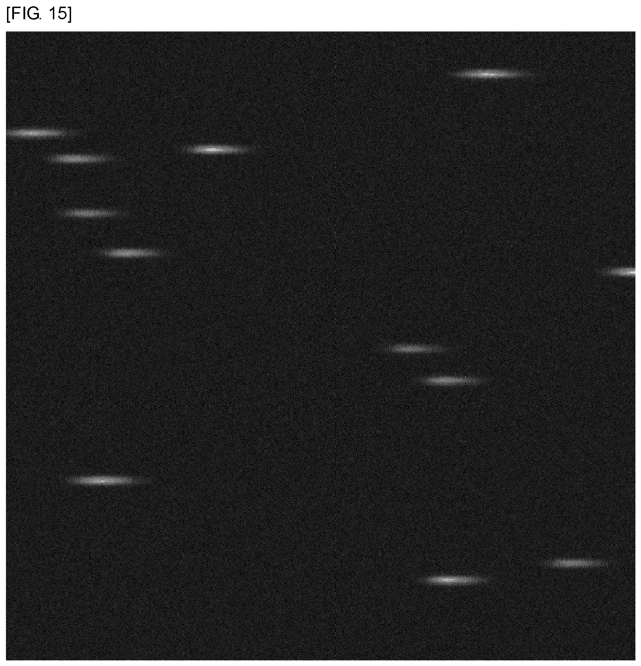

FIG. 15 illustrates observing fluorescence using only a spectroscopic means (quartz wedge prism with an angle of 7 degrees 41 minutes) without an identification filter. Although there is a slight difference in intensity of each fluorescence, it is difficult to identify the fluorescence with only such a slight difference.

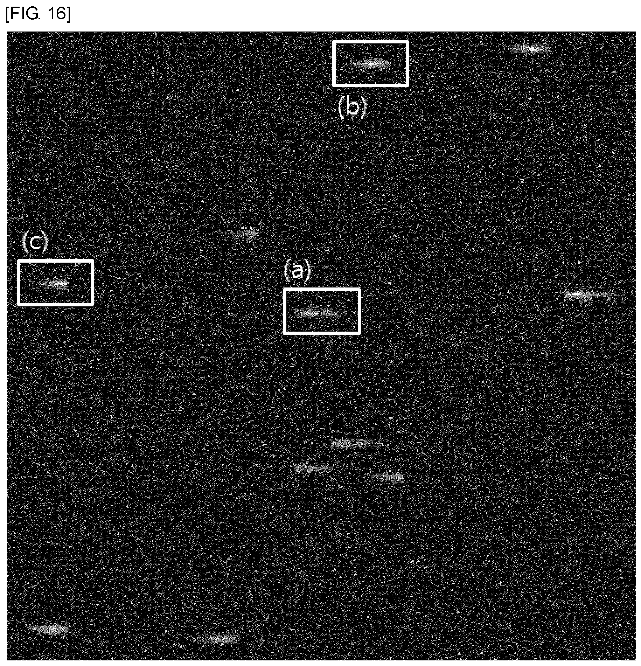

FIG. 16 illustrates observing fluorescence using a spectroscopic means (quartz wedge prism with an angle of 7 degrees 41 minutes) and an identification filter (FF01-680/42-25 filter of Semrock). FIG. 16A illustrates CF647, in which a short wavelength portion was removed and a shape with a cut left side was observed, and FIG. 16B illustrates CF660R, in which the wavelengths on both sides were removed and a shape with both cut sides was observed. In addition, FIG. 16C illustrates CF680R, in which a long wavelength portion was removed and a shape with a cut right side was observed.

As illustrated in FIG. 16, in the present disclosure, the blocked portion of the fluorescence and the relative position and length of the blocked portion are determined using the identification filter to immediately recognize the type of fluorescence, and the fluorescence may be identified on one screen to identify the observed result with improved detection area and sensitivity.

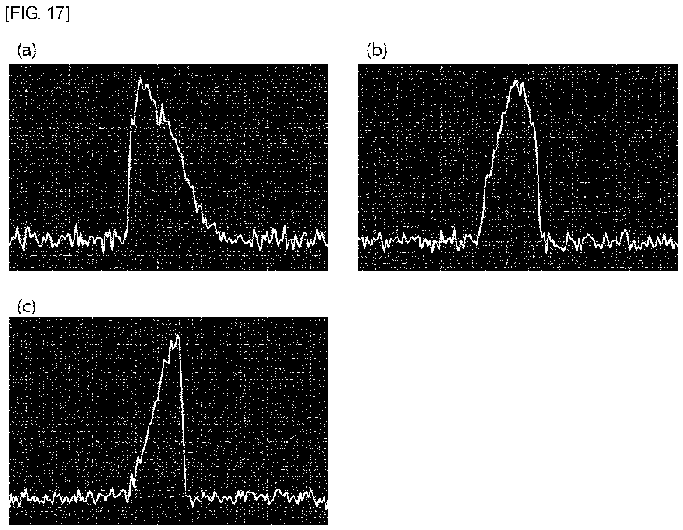

In order to check the blocking effect of the identification filter, each fluorophore passes through the identification filter and the spectroscopic means, and then the spectra were measured. As illustrated in FIG. 17, in the case of (a) CF647, a distribution in which a left side was cut was confirmed, and in the case (b) CF660R, a distribution in which both sides were cut was shown. In addition, in the case of (c) CF670R, a distribution in which a right side was cut was shown. Therefore, it was confirmed that a plurality of fluorophores can be easily identified by using the identification filter and the spectroscopic means of the present disclosure.

As described above, specific parts of the present disclosure have been described in detail, and it will be apparent to those skilled in the art that these specific techniques are merely preferred embodiments, and the scope of the present disclosure is not limited thereto. Therefore, the substantial scope of the present disclosure will be defined by the appended claims.

## Claims

1. A biomarker identification method comprising steps of:
preparing a sample (110) labeled with a fluorophore, wherein:
a capture probe is attached to a substrate,
a biomarker is bound to the capture probe, and
a detection probe is bound to the biomarker and is labeled with a fluorophore;
positioning the sample (110) on an objective unit (100) of a fluorescence microscope;
illuminating the sample (110) using a light source (210);
blocking some of fluorescence spectra emitted from the sample (110) using an identification filter (310);
dispersing the fluorescence with the spectroscopic means (320) for observing a shape of a fluorescence spectrum; and
identifying a type of biomarker in the sample (110) using the observed shape of the fluorescence spectrum
wherein the identifying a type of biomarker in the sample (110) comprises
recognizing a blocked point of the fluorescence spectrum as a cut portion of the observed shape of the fluorescence spectrum;
analyzing a relative position and length of the fluorescence spectrum from the blocked point; and
identifying a type of biomarker in the sample (110) using the relative position and length of the fluorescence spectrum.

2. The biomarker identification method of claim 1, wherein the sample (110) labeled with the fluorophore is 2 to 100 types of biomarkers attached with different fluorophores.

3. The biomarker identification method of claim 1, wherein the identification filter (310) blocks at least one of
some of fluorescence spectra having short-wavelength emission peaks in the fluorescence emitted from the sample (110); and
some of fluorescence spectra having long-wavelength emission peaks in the fluorescence emitted from the sample (110).

4. The biomarker identification method of claim 1, wherein the identification filter (310) blocks at least one of
wavelengths of a peak wavelength or shorter of the fluorescence spectra having the short-wavelength emission peaks in the fluorescence emitted from the sample (110); and
wavelengths of a peak wavelength or longer of the fluorescence spectra having the long-wavelength emission peaks in the fluorescence emitted from the sample (110).

5. The biomarker identification method of claim 1, wherein the fluorescence emitted from the sample (110) has 2 to 100 emission peaks, and some or all of the emission spectra having each spectrum overlap with each other.

6. The biomarker identification method of claim 1, wherein fluorophores included in the sample (110) are simultaneously excited with one or more excitation light.

7. The biomarker identification method of claim 1, wherein 2 to 100 types of detection probes are present on the surface of the substrate.

8. The biomarker identification method of claim 1, wherein the detection probe is labeled with at least one type of fluorophore, and
the biomarker bound to the detection probe has one or more emission peaks.

9. The biomarker identification method of claim 1, wherein the dispersed fluorescence by the spectroscopic means (320) is observed in an elliptical shape with one or both cut sides.

## Patentansprüche

1. Verfahren zur Identifizierung von Biomarkern, umfassend die folgenden Schritte:
Herstellen einer mit einem Fluorophor markierten Probe (110), wobei:
eine Fangsonde an ein Substrat gebunden ist,
ein Biomarker an die Fangsonde gebunden ist und
eine Detektionssonde an den Biomarker gebunden und mit einem Fluorophor markiert ist;
Positionieren der Probe (110) auf einer Objektiveinheit (100) eines Fluoreszenzmikroskops;
Beleuchten der Probe (110) unter Verwendung einer Lichtquelle (210);
Blockieren eines Teils der von der Probe (110) emittierten Fluoreszenzspektren unter Verwendung eines Identifizierungsfilters (310);
Aufspalten der Fluoreszenz mit den spektroskopischen Mitteln (320) zur Beobachtung einer Form eines Fluoreszenzspektrums; und
Identifizieren einer Art von Biomarker in der Probe (110) unter Verwendung der beobachteten Form des Fluoreszenzspektrums,
wobei das Identifizieren einer Art eines Biomarkers in der Probe (110) umfasst:
das Erkennen eines blockierten Punktes des Fluoreszenzspektrums als abgeschnittenen Abschnitt der beobachteten Form des Fluoreszenzspektrums;
das Analysieren einer relativen Position und Länge des Fluoreszenzspektrums ausgehend von dem blockierten Punkt; und
das Identifizieren einer Art des Biomarkers in der Probe (110) unter Verwendung der relativen Position und Länge des Fluoreszenzspektrums.

2. Verfahren zur Identifizierung von Biomarkern nach Anspruch 1, wobei die mit dem Fluorophor markierte Probe (110) aus 2 bis 100 Arten von Biomarkern besteht, an die unterschiedliche Fluorophore gebunden sind.

3. Verfahren zur Identifizierung von Biomarkern nach Anspruch 1, wobei der Identifizierungsfilter (310) mindestens eines der folgenden blockiert:
einige der Fluoreszenzspektren mit kurzwelligen Emissionspeaks in der von der Probe (110) emittierten Fluoreszenz; und
einige der Fluoreszenzspektren mit langwelligen Emissionspeaks in der von der Probe (110) emittierten Fluoreszenz.

4. Verfahren zur Identifizierung von Biomarkern nach Anspruch 1, wobei der Identifizierungsfilter (310) mindestens eine der folgenden blockiert:
Wellenlängen einer Spitzenwellenlänge oder kürzer, der Fluoreszenzspektren mit den kurzwelligen Emissionsspitzen in der von der Probe (110) emittierten Fluoreszenz; und
Wellenlängen einer Spitzenwellenlänge oder länger, der Fluoreszenzspektren mit den langwelligen Emissionsspitzen in der von der Probe (110) emittierten Fluoreszenz.

5. Verfahren zur Identifizierung von Biomarkern nach Anspruch 1, wobei die von der Probe (110) emittierte Fluoreszenz 2 bis 100 Emissionspeaks aufweist und sich einige oder alle der Emissionsspektren, die jedes Spektrum aufweist, gegenseitig überlappen.

6. Verfahren zur Identifizierung von Biomarkern nach Anspruch 1, wobei die in der Probe (110) enthaltenen Fluorophore gleichzeitig mit einem oder mehreren Anregungslichtstrahlen angeregt werden.

7. Verfahren zur Identifizierung von Biomarkern nach Anspruch 1, wobei auf der Oberfläche des Substrats 2 bis 100 Arten von Detektionssonden vorhanden sind.

8. Verfahren zur Identifizierung von Biomarkern nach Anspruch 1, wobei die Detektionssonde mit mindestens einer Art von Fluorophor markiert ist, und
der an die Detektionssonde gebundene Biomarker einen oder mehrere Emissionspeaks aufweist.

9. Verfahren zur Identifizierung von Biomarkern nach Anspruch 1, wobei die durch die spektroskopischen Mittel (320) erfasste gestreute Fluoreszenz in einer elliptischen Form mit einer oder beiden abgeschnittenen Seiten beobachtet wird.

## Revendications

1. Procédé d'identification de biomarqueur comprenant les étapes consistant à :
préparer un échantillon (110) marqué avec un fluorophore, où :
une sonde de capture est fixée à un substrat,
un biomarqueur est lié à la sonde de capture, et
une sonde de détection est liée au biomarqueur et est marquée avec un fluorophore ;
positionner l'échantillon (110) sur une unité d'objectif (100) d'un microscope à fluorescence ;
éclairer l'échantillon (110) en utilisant une source de lumière (210) ;
bloquer certains des spectres de fluorescence émis par l'échantillon (110) en utilisant un filtre d'identification (310) ;
disperser la fluorescence avec le moyen spectroscopique (320) pour observer une forme d'un spectre de fluorescence ; et
identifier un type de biomarqueur dans l'échantillon (110) en utilisant la forme observée du spectre de fluorescence,
où l'identification d'un type de biomarqueur dans l'échantillon (110) comprend
la détection d'un point bloqué du spectre de fluorescence en tant que partie découpée de la forme observée du spectre de fluorescence ;
l'analyse d'une position et d'une longueur relatives du spectre de fluorescence à partir du point bloqué ; et
l'identification d'un type de biomarqueur dans l'échantillon (110) en utilisant la position et la longueur relatives du spectre de fluorescence.

2. Procédé d'identification de biomarqueur selon la revendication 1, où l'échantillon (110) marqué avec le fluorophore est de 2 à 100 types de biomarqueurs fixés avec différents fluorophores.

3. Procédé d'identification de biomarqueur selon la revendication 1, où le filtre d'identification (310) bloque au moins l'un de
certains des spectres de fluorescence ayant des pics d'émission de courte longueur d'onde dans la fluorescence émise par l'échantillon (110) ; et
certains des spectres de fluorescence ayant des pics d'émission de longue longueur d'onde dans la fluorescence émise par l'échantillon (110).

4. Procédé d'identification de biomarqueur selon la revendication 1, où le filtre d'identification (310) bloque au moins l'un de
longueurs d'onde d'une longueur d'onde de pic ou plus courte des spectres de fluorescence ayant les pics d'émission de courte longueur d'onde dans la fluorescence émise par l'échantillon (110) ; et
longueurs d'onde d'une longueur d'onde de pic ou plus longue des spectres de fluorescence ayant les pics d'émission de longue longueur d'onde dans la fluorescence émise par l'échantillon (110).

5. Procédé d'identification de biomarqueur selon la revendication 1, où la fluorescence émise par l'échantillon (110) a 2 à 100 pics d'émission, et certains ou la totalité des spectres d'émission ayant chaque spectre se chevauchent les uns les autres.

6. Procédé d'identification de biomarqueur selon la revendication 1, où les fluorophores inclus dans l'échantillon (110) sont excités simultanément avec une ou plusieurs lumières d'excitation.

7. Procédé d'identification de biomarqueur selon la revendication 1, où 2 à 100 types de sondes de détection sont présents sur la surface du substrat.

8. Procédé d'identification de biomarqueur selon la revendication 1, où la sonde de détection est marquée avec au moins un type de fluorophore, et
le biomarqueur lié à la sonde de détection a un ou plusieurs pics d'émission.

9. Procédé d'identification de biomarqueur selon la revendication 1, où la fluorescence dispersée par le moyen spectroscopique (320) est observée sous une forme elliptique avec un ou les deux côtés découpés.
